# EUROPÄISCHE PATENTSCHRIFT

(11) **EP 2 591 746 B1**
(45) Veröffentlichungstag und Bekanntmachung des Hinweises auf die Patenterteilung: **11.05.2016**
(21) Anmeldenummer: 11188212.2
(22) Anmeldetag: 08.11.2011
(51) Int. Cl.: A61C 1/06, A61B 17/16

(54) **Elektromotor zum Antrieb eines medizinischen, insbesondere dentalen, Instruments**
Electric motor for driving a medical, in particular dental instrument
Moteur électrique destiné à l'entraînement d'instruments médicaux, notamment dentaires

(43) Veröffentlichungstag der Anmeldung: 15.05.2013
(73) Patentinhaber: W & H Dentalwerk Bürmoos GmbH, 5111 Bürmoos (AT)
(72) Erfinder: Pruckner, Christian, 1190 Wien (AT); Mangelberger, Michael, 5113 St. Georgen (AT); Baier, Christof, 5111 Bürmoos (AT); Stephan, Waldemar, 58239 Schwerte (DE)

(56) Entgegenhaltungen:
- EP-A1- 2 073 349
- EP-A2- 0 788 779
- JP-A- 2003 324 871
- US-A- 3 801 843

## Beschreibung

Die vorliegende Erfindung bezieht sich auf einen Elektromotor zum Antrieb eines medizinischen, insbesondere dentalen, Instruments nach dem Oberbegriff des Anspruchs 1.

Ein derartiger Elektromotor dient zum Antrieb medizinischer, insbesondere dentaler, Instrumente. Die Instrumente können hierbei als medizinische, insbesondere dentale, Hand- oder Winkelstücke oder als medizinische, insbesondere dentale Werkzeuge, welche bevorzugt zur Bearbeitung von hartem oder weichem Gewebe oder zum Einbringen von Implantaten dienen, ausgebildet sein. Bevorzugt werden die medizinischen Werkzeuge mittels der Hand- oder Winkelstücke mit einer medizinischen, insbesondere dentalen, Antriebseinheit verbunden und durch den in der Einheit angeordneten Elektromotor angetrieben. Während des Betriebs der medizinischen Instrumente sind diese sowie die Hand- oder Winkelstücke mit Arbeitsmedien, wie zum Beispiel Sprayluft oder Spraywasser zur Kühlung sowie mit Daten und/ oder Energie, insbesondere zur Erkennung der Instrumente und/ oder der Hand- und Winkelstücke, zu versorgen. Diese Medien sowie Betriebsmedien für den Elektromotor, insbesondere elektrische Energie und/ oder Fluide zur Kühlung des Motors, werden insbesondere von einer dentalen Einheit bereit gestellt und mittels einer Versorgungsleitung über eine erste Kupplungsvorrichtung der Antriebseinheit zugeführt. Um die Arbeitsmedien schließlich den medizinischen Instrumenten und/ oder den Hand- oder Winkelstücken, welche über eine zweite Kupplungsvorrichtung mit der Antriebseinheit verbunden sind, insbesondere der Hand- oder Winkelstückköpfe, zu zuführen, weist die Antriebseinheit zumindest eine Medienleitung auf, welche sich durch den Elektromotor, insbesondere von der ersten zur zweiten Kupplungsvorrichtung, erstreckt.

Der Elektromotor selbst ist bevorzugt durch eine Rotoreinheit mit zumindest einem auf einer Welle gelagerten Rotormagneten, welche drehbar in einem Statorpaket mit vorzugsweise mehreren Statorwicklungen gelagert ist, ausgebildet. Das Statorpaket selbst besteht vorzugsweise aus mehreren Statorblechen.

Im Stand der Technik ist es üblich die zumindest eine Medienleitung zur Versorgung der medizinischen Instrumente und/ oder der Hand- oder Winkelstücke mit Arbeitsmedien zwischen dem Motorgehäuse und der darin aufgenommenen Motorkomponenten, insbesondere zwischen Gehäuse und dem Statorpaket des Elektromotors, anzuordnen.

Aus der EP 0 788 779 A2 ist eine weitere Lösung bekannt, um zumindest eine Medienleitung platzsparend durch den Elektromotor zu führen. Hierzu wird die zumindest eine Medienleitung insbesondere direkt durch das Statorpaket, zwischen Rotor und Stator oder durch die Statorwicklung geführt.

Zur Kühlung des Elektromotors, insbesondere dessen Statorpakets, durch ein, vorzugsweise von der dentalen Einheit bereitgestellten, Kühlmedium, auf Grund des Stromflusses in den Statorwicklungen, was eine Erwärmung und somit Leistungsminderung des Motor zur Folge hat, sind im Stand der Technik ebenfalls mehrere Lösungen bekannt.

Die JP 2003324871 A offenbart eine Motoranordnung bei der Kühlfluid an der Außenseite der Statorpakete, insbesondere an den ringförmigen Statorblechen, welche im Zusammenschluss eine zylinderförmige Oberfläche bilden, vorbei geleitet wird. Hierzu weist der Stator oder das Gehäuse des Motors eine oder mehrere Durchgangsbohrungen auf.

Des Weiteren ist es üblich das Kühlfluid direkt durch die Statorpakete, insbesondere durch einen durch die mehreren Statorbleche gebildeten Fluidkanal im Inneren des Statorpakets, durch den Elektromotor hindurch zuleiten.

Die US 3801843 offenbart einen Motor mit einem Rotor und einem Stator sowie mehreren Wärmeleitungen, welche sich durch den Stator erstrecken. Die Wärmeleitungen sind mit einem Kühlmittel befüllt. Ein neben dem Stator und in dem Motor angeordneter Kondensorabschnitt der Wärmeleitungen, welcher mehrere Kühlrippen aufweist, dient zum Abkühlen des in den Leitungen eingeschlossenen Kühlmittels.

Als nachteilig der im Stand der Technik bekannten Ausgestaltungen, insbesondere zur platzsparenden Anordnung der Medienleitungen in den Elektromotoren, erweist sich die Erwärmung der zumindest einen durch den Motor geführten Medienleitung, insbesondere der damit verbundene Wärmeeintrag auf das darin geleitete Arbeitsmedium für das medizinische, insbesondere dentale, Instrument und/ oder Hand- oder Winkelstück, insbesondere durch den Stator des Elektromotors. Das durch die zumindest eine Medienleitung geführte Arbeitsmedium, insbesondere Fluid, dient insbesondere zur Kühlung der mit den Motoren verbundenen medizinischen Instrumente und/ oder Hand- oder Winkelstücke. Somit ist eine Erwärmung dieser Medien unerwünscht, da hiermit eine Verminderung der Kühlleistung des Arbeitsmediums verbunden ist.

Einen weiteren Nachteil der im Stand der Technik bekannten Ausführungsformen zur Kühlung der Statorpakete stellen die damit erzielten geringen Kühlleistungen dar. Durch das Vorbeileiten der Kühlfluide an den Außenseiten der, insbesondere ringförmigen, Statorbleche, welche im Zusammenschluss Statorpakete mit zylinderförmigen Oberflächen bilden, sowie durch das Hindruchleiten der Fluide durch, insbesondere rohrförmige, Fluidkanäle im Inneren der Statorpakete, erfolgt bisher bei allen Ausführungsformen nur eine limitierte Wärmeableitung von dem Statorpaket.

Der vorliegenden Erfindung liegt daher die Aufgabe zugrunde bei einem Elektromotor zum Antrieb eines medizinischen, insbesondere dentalen, Instruments die Wärmeableitung vom Statorpaket zu steigern und gleichzeitig einen Wärmeübertrag von dem Statorpaket auf die zumindest eine durch den Motor geführte Medienleitung zu vermeiden.

Gemäß der Erfindung weist der Elektromotor zum Antrieb eines medizinischen, insbesondere dentalen, Instruments, ein Gehäuse zur Aufnahme einer Rotoreinheit, welche drehbar in einem in dem Gehäuse aufgenommenen Statorpaket mit mehreren Statorblechen gelagert ist, sowie wenigstens eine durch den Elektromotor von einem Einlass bis zu einem Auslass verlaufende Medienleitung zur Zuführung eines Fluids, Daten und/ oder Energie zu dem medizinischen, insbesondere dentalen, Instrument, auf, wobei die zumindest eine Medienleitung durch ein Leitungsrohr gebildet ist in dem die Medien führbar sind und zumindest ein Teil der Statorbleche jeweils zumindest eine Kühllamelle mit einer Aussparung und/ oder zumindest zwei voneinander beabstandete Kühllamellen aufweist und wobei die zumindest eine Medienleitung zumindest teilweise in der Aussparung und/ oder zwischen den zumindest zwei voneinander beabstandete Kühllamellen angeordnet ist, um so zwischen der Medienleitung und der Kühllamelle einen Kühlmittelleitungsabschnitt zu bilden, welcher von einem Kühlmedium durchströmbar ist, so dass mittels des Kühlmediums gleichzeitig das Statorpaket und die zumindest eine Medienleitung kühlbar sind.

Zumindest ein Teil der Statorbleche weist jeweils zumindest eine Kühllamelle mit einer Aussparung auf, um einen Kühlmittelleitungsabschnitt zu bilden, in dem die zumindest eine Medienleitung zumindest teilweise angeordnet ist. Hierbei sind die Statorbleche mit der zumindest einen Kühllamelle in axialer Richtung zur Rotationsachse des Motors bevorzugt parallel angeordnet, so dass die mehreren Kühllamellen, insbesondere deren Aussparungen den Kühlmittelleitungsabschnitt bilden, wobei insbesondere einem Statorblech mit Kühllamelle ein Statorblech ohne Lamelle folgt. Die Aussparung in der zumindest einen Kühllamelle eines Statorblechs ist vorzugsweise durch eine rillenförmige Vertiefung an der Außenseite der Kühllamelle gebildet, um die zumindest eine Medienleitung zumindest teilweise aufzunehmen. Zur gänzlichen Aufnahme der Medienleitung in der Kühllamelle ist die Aussparung bevorzugt durch eine Bohrung in der Lamelle ausgebildet. Sowohl die rillenförmige Vertiefung als auch die Bohrung weisen einen größeren Querschnitt als die darin aufgenommene Medienleitung auf, um so einen Kühlmittelleitungsabschnitt zwischen Kühllamelle und Medienleitung zu bilden.

Zumindest ein Teil der Statorbleche weist jeweils zumindest zwei von einander beabstandete Kühllamellen auf, um einen Kühlmittelleitungsabschnitt zu bilden, in dem die zumindest eine Medienleitung zumindest teilweise angeordnet ist. Hierbei können die mehreren Statorbleche des Statorpakets mit Kühllamellen in axialer Richtung zur Rotationsachse des Motors parallel angeordnet sein, so dass auch der dadurch gebildete Kühlmittelleitungsabschnitt parallel zur Rotationsachse verläuft. Bevorzugt verläuft der durch die mehreren die Kühllamellen aufweisenden Statorbleche gebildete Kühlmittelleitungsabschnitt jedoch wendelförmig um die Rotationsachse des Motors. Hierzu sind die die Kühllamellen aufweisenden Statorbleche winkelversetzt zur Rotationsachse des Motors angeordnet. Des Weiteren kann der Kühlmittelleitungsabschnitt in axialer Richtung zur Rotorachse des Motors die Richtung mehrfach ändern. Insbesondere sind die Statorbleche mit den zumindest zwei Kühllamellen derart zueinander versetzt um die Rotationsachse angeordnet, dass die Kühllamellen zweier aufeinander folgender Statorbleche gänzlich nebeneinander angeordnet sind. Die Flächen der Kühllamellen zweier benachbarter Statorbleche berühren sich somit nicht. Der Abstand zwischen den zumindest zwei Kühllamellen eines Statorblechs beträgt hierzu bevorzugt die Breite einer Kühllamelle oder ein Vielfaches, insbesondere ein Zweifaches der Breite einer Kühllamelle. Diese Anordnung der Statorbleche, insbesondere der Kühllamellen, führt zu einem Kühlmittelleitungsabschnitt, welcher wendelförmig um die Rotationsachse des Motors verläuft und eine Breite von einer oder mehreren Kühllamellen aufweist. Bei diesem Ausführungsbeispiel verläuft die zumindest eine Medienleitung ebenfalls parallel oder wendelförmig in dem Kühlmittelleitungsabschnitt um die Rotationsachse des Motors.

Zumindest ein Teil der Statorbleche weist jeweils zumindest eine erste Kühllamelle mit einer Aussparung und zumindest eine davon beabstandete zweite Kühllamelle auf, um eine Kühlmittelleitung mit zwei Abschnitten zu bilden, wobei zumindest in einem der beiden Abschnitte die zumindest eine Medienleitung zumindest teilweise angeordnet ist. Beide Abschnitte sind bevorzugt gemäß den Merkmalen des ersten und zweiten Ausführungsbeispiels ausgebildet. Die vorzugsweise mehreren Medienleitungen können somit parallel und/ oder wendelförmig um die Rotationsachse des Elektromotors in einem der Abschnitte der Kühlmittelleitung angeordnet sein.

Bei allen beschrieben Ausführungsbeispielen ist die zumindest eine Kühllamelle und die zumindest eine Medienleitung bevorzugt am äußeren Umfang der, insbesondere ringförmigen, Statorbleche des Statorpakets angeordnet. Ebenso können diese an der Innenseite der Statorbleche angebracht sein.

Die zumindest eine Medienleitung zur Zuführung von Fluiden, Daten und/ oder Energie zu dem medizinischen, insbesondere dentalen, Instrument, welche sich durch den Motor erstreckt, ist durch ein Leitungsrohr gebildet, in dem die Medien, insbesondere Fluide, Daten und/ oder Energie führbar sind. Das Leitungsrohr ist hierzu hohl ausgebildet und bildet einen eigenen von dem Kühlmittelleitungsabschnitt getrennten Raum. Hierdurch ist es möglich das Arbeitsmedium für das medizinische Instrument getrennt von dem Medium zur Kühlung des Statorpakets durch den Motor zu führen und/ oder unterschiedliche Medien als Arbeitsmedium und als Kühlmedium zu verwenden und/ oder Medien mit unterschiedlichen Parametern, z.B. unterschiedlichen Fließgeschwindigkeiten oder Drücken, zu verwenden. Bevorzugt ist das Leitungsrohr starr, insbesondere formstabil ausgebildet. In einer weiteren Variante, insbesondere zur einfachen Montage in einem wendelförmigen Kühlmittelleitungsabschnitt, ist es möglich das Rohr flexibel auszugestalten. Bevorzugt ist das Leitungsrohr aus einem Metall oder Kunststoff gefertigt.

Gemäß einem Ausführungsbeispiel einer medizinischen, insbesondere dentalen, Antriebseinheit weist diese zum Antrieb eines über eine erste Kupplungsvorrichtung mit der Antriebseinheit verbundenen medizinischen, insbesondere dentalen, Hand- oder Winkelstücks, einen Elektromotor nach einem der vorherigen Ausführungsbeispiele auf, wobei die Antriebseinheit eine zweite Kupplungsvorrichtung zur Zuführung von Fluiden, Daten und/ oder Energie umfasst.

Gemäß einem Ausführungsbeispiel eines medizinischen, insbesondere dentalen, Hand- oder Winkelstücks weist dieses einen Elektromotor oder eine Antriebseinheit zum Antrieb eines über eine Werkzeugaufnahme aufgenommenes medizinisches, insbesondere dentales, Instrument nach einem der vorherigen Ausführungsbeispiele auf.

Der vorliegende Elektromotor zum Antrieb eines medizinischen, insbesondere dentalen, Instruments zeichnet sich durch eine Reihe erheblicher Vorteile aus.

Durch die gleichzeitige Kühlung des Statorpakets und der zumindest einen durch den Elektromotor verlaufenden Medienleitung mittels des durch den Kühlmittelleitungsabschnitt geleitete Kühlmediums wird ein Wärmeübertrag von dem Statorpaket auf das in der Medienleitung geführte Arbeitsmedium für das medizinische, insbesondere dentale, Instrument und/ oder Hand- oder Winkelstück vermieden oder zumindest verringert.

Des Weiteren wird durch die Anordnung von Kühllamellen zumindest an einem Teil der Statorbleche des Statorpakets die Oberfläche des Pakets vergrößert. Dies bewirkt eine bessere Wärmeableitung von dem Statorpaket durch das an dem Statorpaket vorbei geleitete Kühlmedium.

Ebenso wird durch die zusätzlichen Kühllamellen der Querschnitt des Statorpakets vergrößert, was eine Verbesserung der elektromagnetischen Eigenschaften des Statorpakets zur Folge hat.

Im Rahmen der Erfindung versteht es sich selbstverständlich, dass der Elektromotor zum Antrieb eines medizinischen, insbesondere dentalen, Instruments nicht auf das oben angeführte Beispiel beschränkt ist. Vielmehr kann der Elektromotor unterschiedlich aufgebaut sein. Insbesondere kann die Rotoreinheit statt den Rotormagneten eine oder mehrere Wicklungen und das Statorpaket einen oder mehrere Magnete aufweisen.
Nachfolgend wird die Erfindung anhand mehrerer Ausführungsbeispiele und in Verbindung mit den beigefügten Zeichnungen erläutert.

Dabei zeigt:
Figur 1 eine medizinische, insbesondere dentale, Antriebseinheit, aufweisend einen Elektromotor zum Antrieb eines medizinischen, insbesondere dentalen, Instruments gemäß der vorliegenden Erfindung,
Figur 2 ein erstes Ausführungsbeispiel des Statorpakets des Elektromotors mit mehreren Statorblechen, wobei ein Teil der Statorbleche Kühllamellen aufweist, welche Aussparungen, insbesondere Bohrungen, zur Aufnahme der zumindest einen Medienleitung umfassen,
Figur 3 ein zweites Ausführungsbeispiel des Statorpakets, wobei die Kühllamellen der Statorbleche zur Rotationsachse des Elektromotors winkelversetzt angeordnet sind,
Figur 4 ein drittes Ausführungsbeispiel des Statorpakets mit mehreren Statorblechen und Kühllamellen, wobei die Bleche derart winkelversetzt zur Rotationsachse zueinander angeordnet sind, dass die Kühllamellen zweier aufeinanderfolgende Statorbleche nebeneinander angeordnet sind,
Figur 5 ein viertes Ausführungsbeispiel des Statorpakets, wobei die Aussparungen in den Kühllamellen durch rillenförmige Vertiefungen an den Außenseiten der Lamellen ausgebildet sind,
Figur 6 ein fünftes Ausführungsbeispiel des Statorpakets mit einer Anordnung der Kühllamellen an der Innenseite der ringförmigen Statorbleche;

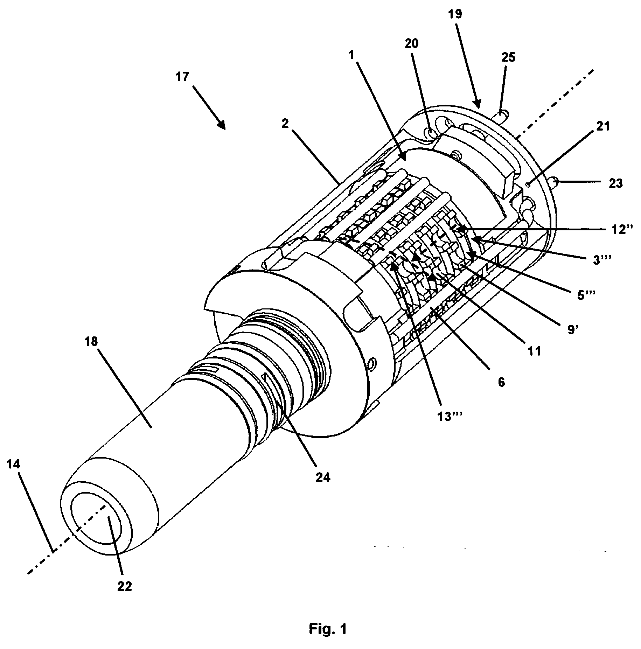

Die in Figur 1 dargestellte medizinische, insbesondere dentale, Antriebseinheit 17 weist einen in einem Motorgehäuse 2 angeordneten Elektromotor 1 auf, welcher zum Antrieb eines medizinischen, insbesondere dentalen, Instruments, das mittels eines medizinischen, insbesondere dentalen, Hand- oder Winkelstücks über eine erste Kupplungsvorrichtung 18 mit der Antriebseinheit 17 kuppelbar ist, auf. Alternativ kann die Antriebseinheit 17 oder der Elektromotor 1 Teil des medizinischen, insbesondere dentalen, Hand- oder Winkelstücks sein. An einem zweiten Ende umfasst die Einheit 17 eine zweite Kupplungsvorrichtung 19, welche, insbesondere über einen Versorgungsschlauch, mit einer dentalen Einheit verbindbar ist, um dem Motor 1 Betriebsmedien, insbesondere elektrische Energie und/ oder Medien zur Kühlung des Motors 1, und dem mit der Antriebseinheit 17 verbundenen medizinischen Instrument sowie Hand- oder Winkelstück Arbeitsmedien zuzuführen. Hierzu weist die Einheit 17 an der zweiten Kupplungsvorrichtung 19 zumindest einen elektrischen Kontakt 25, zumindest einen Kühlmitteleinlass 20 sowie einen Arbeitsmitteleinlass 23 auf. Der Einlass 23 ist bevorzugt durch eine Medienleitung 6, insbesondere durch ein Leitungsrohr, gebildet, welche sich durch den Elektromotor, insbesondere durch dessen Statorpaket 3"', bis zu einem Arbeitsmittelauslass 24 an der ersten Kupplungsvorrichtung 18 erstreckt. Im Inneren des Vorsprungs 18 ist zumindest ein Teil der Rotorwelle der Rotoreinheit zum Antrieb einer Arbeitswelle des mit der Einheit 17 verbundenen medizinischen Hand- oder Winkelstücks drehbar um die Rotationsachse 14 gelagert.

Um den Motor 1 mittels des durch den Einlass 20 eingebrachten Kühlmediums zu kühlen, weist dieser ein Statorpaket 3"' gemäß eines vierten Ausführungsbeispiels der Erfindung auf. Hierbei umfassen die Statorbleche 5'" des Statorpakets 3'" jeweils mehrere Kühllamellen 9', um so die Oberfläche des Statorpakets 3'" zu vergrößern und einen, insbesondere wendelförmig, um den Stator 3'" verlaufenden Kühlmittelleitungsabschnitt 13"' zu bilden. Des Weiteren weisen die Kühllamellen 9' Aussparungen, insbesondere rillenförmige Vertiefungen 11 auf, um die zumindest eine Medienleitung 6 zumindest teilweise in den Kühllamellen 9' aufzunehmen und zwischen der Leitung 6 und der Aussparung einen weiteren Kühlmittelabschnitt 12" zur gleichzeitigen Kühlung der Medienleitung 6 und des Statorpakets 3'" zu bilden. Mittels des ersten und zweiten Kühlmittelauslass 21, 22 kann das Kühlmedium nach der Umströmung des Statorpakets 3'" aus dem Motorgehäuse 2, insbesondere aus der Antriebseinheit austreten.

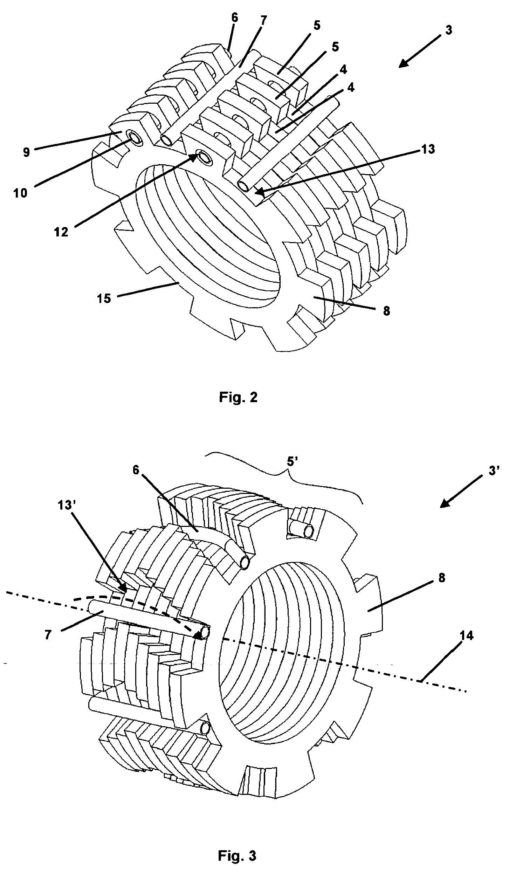

In Figur 2 ist ein erstes Ausführungsbeispiel des Statorpakets 3 des Elektromotors 1 abgebildet. Hierbei weist zumindest ein Teil der Statorbleche 4, 5 jeweils zumindest eine Kühllamelle 8, 9 auf. Je nach Anzahl der durch den Motor zu führenden Medienleitungen 6, 7 weisen diese Kühllamellen 9 Aussparungen, insbesondere Bohrung 10, auf, welche einen größeren Querschnitt als die darin aufgenommene Medienleitung 6 umfassen, um so einen Kühlmittelleitungsabschnitt 12 zwischen der Kühllamelle 9 und der Medienleitung 6 zu bilden. Bevorzugt sind die Statorbleche 5 mit der zumindest einen Kühllamelle 8, 9 am äußeren Umfang 15 in axialer Richtung zur Rotationsachse des Motors parallel angeordnet. Zusätzlich wird durch die voneinander beabstandete Anordnung von zumindest zwei Kühllamellen 8, 9 an einem Statorblech 5 ein weiterer ebenfalls zur Rotationsachse paralleler Kühlmittelleitungsabschnitt 13 gebildet. Um die Oberfläche des Statorpakets möglichst groß zu gestalten, sind die Statorbleche 4, 5 des Statorpakets derart angeordnet, dass insbesondere einem Statorblech 5 mit zumindest einer Kühllamelle 8, 9 ein Statorblech 4 ohne Lamellen folgt.

In Figur 3 ist ein zweites Ausführungsbeispiel des Statorpakets 3' gezeigt, wobei die Kühllamellen 8 der Statorbleche 5' zur Rotationsachse 14 des Elektromotors winkelversetzt angeordnet sind. Insbesondere sind die Kühllamellen 8 zweier benachbarter Statorbleche 5' versetzt, aber einander zumindest teilweise überlagernd angeordnet. Hierdurch verläuft der durch die zumindest zwei voneinander beabstandet angeordneten Kühllamellen 8 gebildete Kühlmittelleitungsabschnitt 13' wendelförmig um die Rotationsachse 14 des Motors. Des Weiteren kann der Kühlmittelleitungsabschnitt 13' in axialer Richtung zur Rotorachse 14 des Motors die Richtung mehrfach ändern. Der Abstand zwischen den zumindest zwei Kühllamellen eines Statorblechs beträgt bevorzugt die Breite einer Kühllamelle 8. Zumindest weist der Querschnitt des Kühlmittelleitungsabschnitts 13' ein größeres Maß als die darin angeordnete Medienleitung 6, 7 auf, so dass gleichzeitig die Kühllamellen 8 und die Medienleitung 6, 7 von dem Kühlfluid des Motors umströmbar sind. Die Medienleitung 7 erstreckt sich hierzu bevorzugt ebenfalls wendelförmig um die Rotationsachse 14 des Elektromotors.

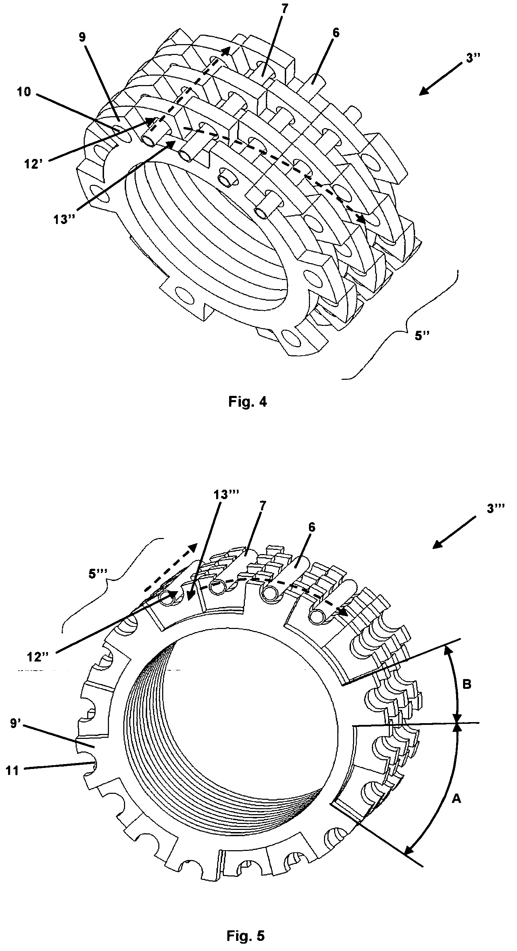

Figur 4 zeigt ein drittes Ausführungsbeispiel des Statorpakets 3". Hierbei weisen alle Statorbleche 5" des Statorpaktes 3" zumindest eine Kühllamelle 9 mit einer Aussparung, insbesondere mit einer Bohrung 10, und zumindest eine davon beabstandete zweite Kühllamelle 9 auf, um zwei Kühlmittelleitungsabschnitte 12', 13" zu bilden. Der erste Abschnitt 12' wird, wie aus dem ersten Ausführungsbeispiel bekannt, durch die Aussparungen, insbesondere Bohrungen 10, in den Kühllamellen 9 gebildet, welche einen größeren Querschnitt als die darin aufgenommene Medienleitung 6, 7 umfassen, um so einen Kühlmittelleitungsabschnitt 12' zwischen Kühllamelle und Medienleitung 6, 7 zu bilden. Dieser erste Kühlmittelleitungsabschnitt 12' erstreckt sich vorzugsweise parallel zur Rotationsachse 14 des Motors durch das Statorpaket 3". Im Unterschied zum ersten Ausführungsbeispiel sind die Statorbleche 5" des Statorpakets 3" zur Rotationsachse 14 des Elektromotors winkelversetzt angeordnet. Insbesondere sind die Statorbleche 5" derart zueinander versetzt angeordnet, dass die Kühllamellen 9 zweier aufeinander folgender Statorbleche 5" gänzlich nebeneinander angeordnet sind. Die Flächen der Kühllamellen 9 berühren einander somit nicht oder nur sehr gering, z.B. unter 10% der Fläche. Der Abstand zweier Kühllamellen 9 eines Statorblechs 5" beträgt vorzugsweise ein Zweifaches der Breite einer Kühllamelle 9, wodurch ein zweiter Kühlmittelleitungsabschnitt 13" gebildet wird, welcher sich wendelförmig um die Rotationsachse 14 des Motors erstreckt.

In Figur 5 ist ein viertes Ausführungsbeispiel des Statorpakets 3'" gezeigt, wobei die Aussparungen in den Kühllamellen 9' durch rillenförmige Vertiefungen 11 an den Außenseiten der Lamellen 9' ausgebildet sind. Wie in Figur 4 weisen alle Statorbleche 5'" des Statorpaktes 3'" zumindest eine Kühllamelle 9' mit einer Aussparung und zumindest eine davon beabstandete zweite Kühllamelle 9' auf, um zwei Kühlmittelleitungsabschnitte 12", 13"', insbesondere einen ersten zur Rotationsachse des Motors parallelen und einen zweiten sich wendelförmig um den Stator 3"' ersteckenden Abschnitt, zu bilden. Der Abstand A zweier Kühllamellen 9' eines Statorblechs 5'" beträgt bevorzugt ein Zweifaches der Breite B einer Kühllamelle 9'. Der erste Abschnitt 12" wird durch die Aussparungen, insbesondere Vertiefungen 11, in den Kühllamellen 9' gebildet, in welchem vorzugsweise die Medienleitungen 6 und 7 angeordnet sind. Der zweite Abschnitt 13'" ist durch die versetzte Anordnung der Kühllamellen 9' benachbarter Statorbleche 5"' gebildet.

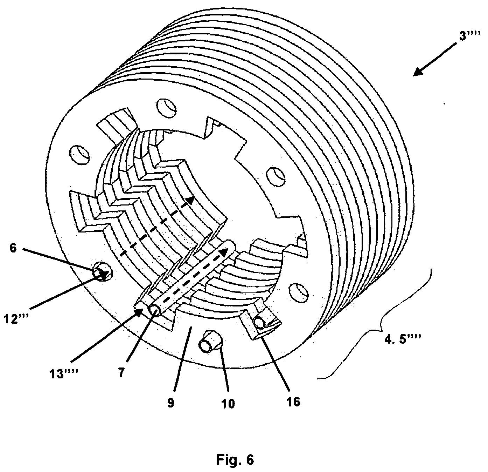

In Figur 6 ist ein fünftes Ausführungsbeispiel des Statorpakets 3"" dargestellt. Hierbei weist zumindest ein Teil der Statorbleche 4, 5"" jeweils zumindest eine Kühllamelle 9 an der Innenseite 16 der im Wesentlichen ringförmigen Statorbleche 5"" auf. In allen Kühllamellen 9 sind Aussparungen, insbesondere Bohrungen 10, angebracht, um einen Kühlmittelleitungsabschnitt 12'" zwischen Kühllamelle 9 und der darin angeordneten Medienleitung 6, 7 zu bilden. Des Weiteren wird durch die Anordnung von zumindest zwei Kühllamellen 9 an einem Statorblech 5"" ein weiterer zur Rotationsachse paralleler Kühlmittelleitungsabschnitt 13"" gebildet. Um die Oberfläche des Statorpakets 3"" möglichst groß zu gestalten, folgt einem Statorblech 5"" mit zumindest einer Kühllamelle 9 ein Statorblech 4 ohne Lamellen.

## Patentansprüche

1. Elektromotor (1) zum Antrieb eines medizinischen, insbesondere dentalen, Instruments, **gekennzeichnet durch** ein Gehäuse (2) zur Aufnahme einer Rotoreinheit, welche drehbar in einem in dem Gehäuse (2) aufgenommenen Statorpaket (3, 3', 3", 3"', 3"") mit mehreren Statorblechen (4; 5, 5', 5", 5"', 5"") gelagert ist, sowie wenigstens eine **durch** den Elektromotor (1) von einem Einlass (23) bis zu einem Auslass (24) verlaufende Medienleitung (6, 7) zur Zuführung eines Fluids, Daten und/ oder Energie zu dem medizinischen, insbesondere dentalen, Instrument, wobei die zumindest eine Medienleitung (6, 7) **durch** ein Leitungsrohr gebildet ist in dem die Medien führbar sind und zumindest ein Teil der Statorbleche (5, 5', 5", 5"', 5"") jeweils zumindest eine Kühllamelle (9, 9') mit einer Aussparung (10, 11) und/ oder zumindest zwei von einander beabstandete Kühllamellen (8, 9, 9') aufweist, und wobei die zumindest eine Medienleitung (6, 7) zumindest teilweise in der Aussparung (10, 11) und/ oder zwischen den zumindest zwei voneinander beabstandeten Kühllamellen (8, 9, 9') angeordnet ist, um so zwischen der Medienleitung (6, 7) und der Kühllamelle (8, 9, 9') einen Kühlmittelleitungsabschnitt (12, 12', 12", 12"'; 13, 13', 13", 13"', 13"") zu bilden, welcher von einem Kühlmedium durchströmbar ist, so dass mittels des Kühlmediums gleichzeitig das Statorpaket (3, 3', 3", 3"', 3"") und die zumindest eine Medienleitung (6, 7) kühlbar sind.

2. Elektromotor (1) zum Antrieb eines medizinischen, insbesondere dentalen, Instruments nach Anspruch 1, **dadurch gekennzeichnet, dass** der Kühlmittelleitungsabschnitt (13', 13", 13"') wendelförmig um die Rotationsachse (14) des Motors verläuft.

3. Elektromotor (1) zum Antrieb eines medizinischen, insbesondere dentalen, Instruments nach Anspruch 1 oder 2, **dadurch gekennzeichnet, dass** der Kühlmittelleitungsabschnitt (13') in axialer Richtung zur Rotorachse (14) des Motors (1) die Richtung mehrfach ändert.

4. Elektromotor (1) zum Antrieb eines medizinischen, insbesondere dentalen, Instruments nach Anspruch 1, **dadurch gekennzeichnet, dass** der Kühlmittelleitungsabschnitt (12, 12', 12", 12"'; 13, 13"") in axialer Richtung zur Rotorachse (14) des Motors (1) parallel angeordnet ist.

5. Elektromotor (1) zum Antrieb eines medizinischen, insbesondere dentalen, Instruments nach einem der vorherigen Ansprüche, **dadurch gekennzeichnet, dass** der Abstand (A) zwischen zwei Kühllamellen (8, 9, 9') eines Statorblechs (5, 5', 5", 5"', 5"") die Breite (B) einer Kühllamelle (8, 9, 9') oder ein Vielfaches, insbesondere ein Zweifaches der Breite (B), einer Kühllamelle (8, 9, 9') beträgt.

6. Elektromotor (1) zum Antrieb eines medizinischen, insbesondere dentalen, Instruments nach einem der vorherigen Ansprüche, **dadurch gekennzeichnet, dass** die Aussparungen in den Kühllamellen (9, 9') durch rillenförmige Vertiefungen (11) an den Außenseiten der Kühllamellen (9') gebildet sind, um die zumindest eine Medienleitung (6, 7) zumindest teilweise aufzunehmen.

7. Elektromotor (1) zum Antrieb eines medizinischen, insbesondere dentalen, Instruments nach einem der vorherigen Ansprüche, **dadurch gekennzeichnet, dass** die Aussparungen in den Kühllamellen (9, 9') durch Bohrungen (10) in den Kühllamellen (9) ausgebildet sind.

8. Elektromotor (1) zum Antrieb eines medizinischen, insbesondere dentalen, Instruments nach einem der vorherigen Ansprüche, **dadurch gekennzeichnet, dass** der Kühlmittelleitungsabschnitt (12, 12', 12", 12"'; 13, 13', 13"") einen größeren Querschnitt als die darin aufgenommene Medienleitung (6, 7) aufweist.

9. Elektromotor (1) zum Antrieb eines medizinischen, insbesondere dentalen, Instruments nach einem der vorherigen Ansprüche, **dadurch gekennzeichnet, dass** das zumindest eine Leitungsrohr starr ausgebildet ist.

10. Elektromotor (1) zum Antrieb eines medizinischen, insbesondere dentalen, Instruments nach einem der vorherigen Ansprüche, **dadurch gekennzeichnet, dass** das zumindest eine Leitungsrohr flexibel ausgebildet ist.

11. Elektromotor (1) zum Antrieb eines medizinischen, insbesondere dentalen, Instruments nach einem der vorherigen Ansprüche, **dadurch gekennzeichnet, dass** die zumindest eine Kühllamelle (8, 9, 9') und die zumindest eine Medienleitung (6, 7) am äußeren Umfang (15) des insbesondere ringförmigen Statorblechs (5, 5', 5", 5"') angeordnet sind.

12. Elektromotor (1) zum Antrieb eines medizinischen, insbesondere dentalen, Instruments nach einem der vorherigen Ansprüche, **dadurch gekennzeichnet, dass** die zumindest eine Kühllamelle (8, 9, 9') und die zumindest eine Medienleitung (6, 7) an der Innenseite (16) des insbesondere ringförmigen Statorblechs (5"") angeordnet sind.

13. Medizinische, insbesondere dentale, Antriebseinheit (17), **gekennzeichnet durch** einen Elektromotor (1) nach einem der Ansprüche 1 bis 12 zum Antrieb eines über eine erste Kupplungsvorrichtung (18) mit der Antriebseinheit (17) verbundenen medizinischen, insbesondere dentalen, Hand- oder Winkelstücks, wobei die Antriebseinheit (17) eine zweite Kupplungsvorrichtung (19) zur Zuführung von Fluiden, Daten und/ oder Energie aufweist.

14. Medizinisches, insbesondere dentales, Hand- oder Winkelstück, **gekennzeichnet durch** einen Elektromotor (1) nach einem der Ansprüche 1 bis 12 zum Antrieb eines über eine Werkzeugaufnahme aufgenommenes medizinisches, insbesondere dentales, Instrument oder **durch** eine medizinische, insbesondere dentale, Antriebseinheit (17) nach Anspruch 13.

## Claims

1. An electric motor (1) for driving a medical, in particular dental, instrument, **characterized by** a housing (2) for holding a rotor unit, which is rotatably supported in a stator packet (3, 3', 3", 3"', 3""), which is accommodated in the housing (2) and has a plurality of stator plates (4; 5, 5', 5", 5"', 5"") and at least one media line (6, 7) running through the electric motor (1) from an inlet (23) to an outlet (24) for supplying a fluid, data and/or energy to the medical, in particular dental, instrument, wherein the at least one media line (6, 7) is formed by a line tube, in which the media can be carried and at least each of a portion of the stator plates (5, 5', 5", 5"', 5"") comprises at least one cooling plate (9, 9') with a recess (10, 11) and/or at least two cooling plates (8, 9, 9') arranged a distance apart from one another, and wherein the at least one media line (6, 7) is arranged at least partially in the recess (10, 11) and/or between the at least two cooling plates (8, 9, 9') arranged a distance apart from one another, to thereby form a coolant line segment (12, 12', 12", 12"'; 13, 13', 13", 13"', 13"") between the media line (6, 7) and the cooling plates (8, 9, 9') through which a cooling medium can flowing, so that by means of the cooling medium, the stator packet (3, 3', 3", 3"', 3"") and the at least one media line (6, 7) can be cooled at the same time.

2. The electric motor (1) for driving a medical, in particular dental, instrument according to Claim 1, **characterized in that** the coolant line segment (13', 13", 13"') runs in a helical pattern around the axis of rotation (14) of the motor.

3. The electric motor (1) for driving a medical, in particular dental, instrument according to Claim 1 or 2, **characterized in that** the coolant line segment (13') changes direction several times in the axial direction relative to the rotor axis (14) of the motor (1).

4. The electric motor (1) for driving a medical, in particular dental, instrument according to Claim 1, **characterized in that** the coolant line segment (12, 12', 12", 12"'; 13, 13"") is arranged in parallel with the rotor axis (14) of the motor (1) in the axial direction.

5. The electric motor (1) for driving a medical, in particular dental, instrument according to any one of the preceding claims, **characterized in that** the distance (A) between two cooling plates (8, 9, 9') of a stator plate (5, 5', 5", 5"', 5"") amounts to the width (B) of a cooling plate (8, 9, 9') or a multiple thereof, in particular twice the width (B) of a cooling plate (8, 9, 9').

6. The electric motor (1) for driving a medical, in particular dental, instrument according to any one of the preceding claims, **characterized in that** the recesses in the cooling plates (9, 9') are formed by groove-type indentations (11) on the outsides of the cooling plates (9') to at least partially accommodate the at least one media line (6, 7).

7. The electric motor (1) for driving a medical, in particular dental, instrument according to any one of the preceding claims, **characterized in that** the recesses in the cooling plates (9, 9') are formed by boreholes (10) in the cooling plates (9).

8. The electric motor (1) for driving a medical, in particular dental, instrument according to any one of the preceding claims, **characterized in that** the coolant line segment (12, 12', 12", 12"'; 13, 13', 13"") has a larger cross section than the media line (6, 7) accommodated in it.

9. The electric motor (1) for driving a medical, in particular dental, instrument according to any one of the preceding claims, **characterized in that** the at least one line pipe is designed to be rigid.

10. The electric motor (1) for driving a medical, in particular dental, instrument according to any one of the preceding claims, **characterized in that** the at least one line pipe is designed to be flexible.

11. The electric motor (1) for driving a medical, in particular dental, instrument according to any one of the preceding claims, **characterized in that** the at least one cooling plate (8, 9, 9') and the at least one media line (6, 7) are arranged on the outer circumference (15) of the stator plate (5, 5', 5", 5"'), which is ring-shaped in particular.

12. The electric motor (1) for driving a medical, in particular dental, instrument according to any one of the preceding claims, **characterized in that** the at least one cooling plate (8, 9, 9') and the at least one media line (6, 7) are arranged on the inside (16) of the stator plate (5""), which is ring-shaped in particular.

13. A medical, in particular dental, drive unit (17), **characterized by** an electric motor (1) according to any one of Claims 1 to 12 for driving a medical, in particular dental, handpiece or contra-angle handpiece that is connected to the drive unit (17) by a first coupling device (18), wherein the drive unit (17) comprises a second coupling device (19) for supplying fluids, data and/or energy.

14. A medical, in particular dental, handpiece or contra-angle handpiece, **characterized by** an electric motor (1) according to any one of Claims 1 to 12, for driving a medical, in particular dental, instrument accommodated via a tool receptacle or by a medical, in particular dental, drive unit (17) according to Claim 13.

## Revendications

1. Moteur électrique (1) pour l'entraînement d'un instrument médical, en particulier dentaire, **caractérisé par** un boîtier (2) pour réceptionner une unité de rotor qui est montée de manière rotative dans un paquet de stator (3, 3', 3", 3"', 3"") réceptionné dans le boîtier (2) avec plusieurs tôles statoriques (4 ; 5, 5', 5", 5"', 5""), ainsi qu'au moins une conduite de milieu (6, 7) s'étendant à travers le moteur électrique (1) depuis une entrée (23) jusqu'à une sortie (24) pour l'amenée d'un fluide, de données et/ou d'énergie vers l'instrument médical, en particulier dentaire, dans lequel l'au moins une conduite de milieu (6, 7) est formée par une conduite dans laquelle les milieux peuvent être véhiculés, et au moins une partie des tôles statoriques (5, 5', 5", 5"', 5"") présentant respectivement au moins une lamelle de refroidissement (8, 9, 9') avec un évidement (10, 11) et/ou au moins deux lamelles de refroidissement (8, 9, 9') espacées l'une de l'autre, et dans lequel l'au moins une conduite de milieu (6, 7) est disposée au moins partiellement dans l'évidement (10, 11) et/ou entre les au moins deux lamelles de refroidissement (8, 9, 9') espacées l'une par rapport à l'autre pour former ainsi, entre la conduite de milieu (6, 7) et la lamelle de refroidissement (8, 9, 9'), un tronçon de conduite d'agent de refroidissement (12, 12', 12", 12'" ; 13, 13', 13", 13"', 13"") qui peut être traversé par un milieu de refroidissement de telle sorte qu'à l'aide du milieu de refroidissement, il est possible de refroidir simultanément le paquet de stator (3, 3', 3", 3"', 3"") et l'au moins une conduite de milieu (6, 7).

2. Moteur électrique (1) pour l'entraînement d'un instrument médical, en particulier dentaire selon la revendication 1, **caractérisé en ce que** le tronçon de conduite d'agent de refroidissement (13', 13", 13"') s'étend en forme de spirale autour de l'axe de rotation (14) du moteur.

3. Moteur électrique (1) pour l'entraînement d'un instrument médical, en particulier dentaire selon la revendication 1 ou 2, **caractérisé en ce que** le tronçon de conduite d'agent de refroidissement (13') change plusieurs fois la direction en direction axiale à l'axe de rotor (14) du moteur (1).

4. Moteur électrique (1) pour l'entraînement d'un instrument médical, en particulier dentaire selon la revendication 1, **caractérisé en ce que** le tronçon de conduite d'agent de refroidissement (12, 12', 12", 12'" ; 13, 13"") est disposé parallèlement en direction axiale par rapport à l'axe de rotor (14) du moteur (1).

5. Moteur électrique (1) pour l'entraînement d'un instrument médical, en particulier dentaire selon l'une des revendications précédentes, **caractérisé en ce que** la distance (A) entre deux lamelles de refroidissement (8, 9, 9') d'une tôle statorique (5, 5', 5", 5"', 5"") est de l'ordre de la largeur (B) d'une lamelle de refroidissement (8, 9, 9') ou de l'ordre d'un multiple de la largeur (B), en particulier de deux fois la largeur (B), d'une lamelle de refroidissement (8, 9, 9').

6. Moteur électrique (1) pour l'entraînement d'un instrument médical, en particulier dentaire selon l'une des revendications précédentes, **caractérisé en ce que** les évidements dans les lamelles de refroidissement (9, 9') sont formés par des creux (11) en forme de rainures sur les côtés extérieurs des lamelles de refroidissement (9') pour réceptionner au moins partiellement l'au moins une conduite de milieu (6, 7).

7. Moteur électrique (1) pour l'entraînement d'un instrument médical, en particulier dentaire selon l'une des revendications précédentes, **caractérisé en ce que** les évidements dans les lamelles de refroidissement (9, 9') sont réalisés par des forures (10) dans les lamelles de refroidissement (9).

8. Moteur électrique (1) pour l'entraînement d'un instrument médical, en particulier dentaire selon l'une des revendications précédentes, **caractérisé en ce que** le tronçon de conduite d'agent de refroidissement (12, 12', 12", 12'" ; 13, 13', 13"") présente une section plus grande que la conduite de milieu (6, 7) qui y est réceptionnée.

9. Moteur électrique (1) pour l'entraînement d'un instrument médical, en particulier dentaire, selon l'une des revendications précédentes, **caractérisé en ce que** l'au moins une conduite est réalisée de manière rigide.

10. Moteur électrique (1) pour l'entraînement d'un instrument médical, en particulier dentaire, selon l'une des revendications précédentes, **caractérisé en ce que** l'au moins une conduite est réalisée de manière flexible.

11. Moteur électrique (1) pour l'entraînement d'un instrument médical, en particulier dentaire, selon l'une des revendications précédentes, **caractérisé en ce que** l'au moins une lamelle de refroidissement (8, 9, 9') et l'au moins une conduite de milieu (6, 7) sont disposées sur le pourtour extérieur (15) de la tôle statorique (5, 5', 5", 5"') en particulier de forme annulaire.

12. Moteur électrique (1) pour l'entraînement d'un instrument médical, en particulier dentaire selon l'une des revendications précédentes, **caractérisé en ce que** l'au moins une lamelle de refroidissement (8, 9, 9") et l'au moins une conduite de milieu (6, 7) sont disposées sur le côté intérieur (16) de la tôle statorique (5"") en particulier de forme annulaire.

13. Unité d'entraînement (17) médicale, en particulier dentaire, **caractérisée par** un moteur électrique (1) selon l'une des revendications 1 à 12 pour l'entraînement d'une pièce à main ou coudée médicale, en particulier dentaire, reliée par l'intermédiaire d'un premier dispositif de couplage (18) à l'unité d'entraînement (17), dans laquelle l'unité d'entraînement (17) présente un deuxième dispositif de couplage (19) pour l'amenée de fluides, de données et/ou d'énergie.

14. Pièce à main ou coudée médicale, en particulier dentaire, **caractérisée par** un moteur électrique (1) selon l'une des revendications 1 à 12 pour l'entraînement d'un instrument médical, en particulier dentaire réceptionné via un logement d'outil, ou par une unité d'entraînement (17) médicale, en particulier dentaire selon la revendication 13.
